# EUROPEAN PATENT SPECIFICATION

(11) **EP 2 313 038 B1**
(45) Date of publication and mention of the grant of the patent: **18.01.2012**
(21) Application number: 09802512.5
(22) Date of filing: 28.07.2009
(51) Int. Cl.: A61F 7/10

(54) **Insole with refreshing or warming effect for user**
Einlegesohle mit Erfrischungs- oder Wärmeeffekt für den Benutzer
Semelle présentant pour l'utilisateur un effet rafraîchissant ou réchauffant

(30) Priority: 31.07.2008 IT FE20080025
(43) Date of publication of application: 27.04.2011
(73) Proprietor: Bordoni, Claudio, 62010 Morrovalle (IT); Funari, Antonio, 63018 Porto Sant'Elpidio (IT)
(72) Inventor: MACINO, Antonio, I-44030 Ferrara (IT)
(74) Representative: Baldi, Claudio
(86) International application number: PCT/EP2009/059774
(87) International publication number: WO 2010/012750

(56) References cited:
- EP-A- 0 608 789
- FR-A- 2 658 415
- GB-A- 102 643
- US-A- 4 422 877
- US-A- 6 102 937
- US-A1- 2004 116 990
- US-A1- 2005 209 663

## Description

The present patent application for industrial invention relates to an article with refreshing or warming effect to be used in the hot or cold season. In particular, the present invention relates to an insole for shoes, it being understood that it can be extended to any other article, such as a clothing item or seat covers and the like.

During the hot season a fresh sensation in some parts of the body may be appreciated, with special reference to parts subject to perspiration, such as the foot. On the contrary, during the cold season, the user may feel the need to warm up some parts of his body.

Products in the form of creams, lotions, balsams, spray and the like are known, which are applied directly on the skin and create a fresh or warm sensation in direct contact with the skin. The efficacy of these products is related with the concentration of active substance they contain, keeping in mind that excessive concentration may burn and cause skin rash. In any case the refreshing or warming effect caused by these products is limited in time. US2004/011699 discloses a thermal therapy pad with variable heat control, comprising a isolating layer, a reactive core, and a plurality of removable layers

FR2658415 disclose a solution for cryotherapy comprising salts, menthol and canfor.

The purpose of the present invention is to eliminate the drawbacks of the technique by devising an article with refreshing or warming effect that prevents the contact of the active substances with the skin.

Another purpose of the present invention is to devise an article with refreshing or warming effect that is characterised by efficacy, efficiency and long duration of the refreshing or warming effect.

Another purpose of the present invention is to devise such an article with refreshing or warming effect that can be worn characterised by versatility for possible application in shoes, clothing items and covers, which is inexpensive and simple to make.

These purposes have been achieved by the invention with the characteristics illustrated in the attached independent claim 1.

Advantageous embodiments of the invention are disclosed from the dependent claims.

The article with refreshing or warming effect to be worn according to the present invention comprises a support that contains a suitable composition to generate a sensation of cool or warmth.

The composition comprises mineral salts mixed with an active substance suitable to generate a sensation of cool or an active substance suitable to generate a sensation of warmth.

The support can consist in sheet material closed to form a bag that contains the composition so that the composition is not in direct contact with the skin.

Advantageously, the support can be soaked with the composition and at least the part of support designed to come in contact with the user is covered with suitable material. This solution is advantageous in case of application in an insole for shoes, in which the support is made of regenerated leather or fabric that is soaked with said solution and coupled with a layer of regenerated leather or fabric without composition designed to come in contact with the foot plant.

Additional characteristics of the invention will appear evident from the following detailed description, which refers to merely illustrative, not limiting embodiments, wherein:
Fig. 1 is a top view of an insole for shoes with warming or refreshing effect according to the present invention;
Fig. 2 is a cross-sectional view along the plane II-II of Fig. 1.

Following is a description of some examples of composition formulations for an article with refreshing effect.

The composition consists in a mixture of salts and active substance with refreshing effect.

Natural menthol (CAS 89-78-1) has proved to be the most suitable active substance with refreshing effect, whereas its derivates that are available on the market, such as lactates, have proved to be inappropriate because they have a low melting point and are transformed in liquids at skin temperature. While searching for possible synergies, mixtures of menthol with balsamic essential oils containing eucalyptol, cypress, rosemary, pine and fresh essential oils containing lavender, lemon and bergamot have been investigated. Also a mixture of menthol and camphor has been investigated. Saline composition has been chosen because it is easy to find and based on poor hygroscopicity, chemical inertia with respect to the other components and stability in association with the support. Sodium chloride (cooking salt), potassium chloride, anhydrous sodium sulphate, anhydrous potassium sulphate, and heptahydrate magnesium sulphate have proved to be suitable. Calcium sulphate dihydrate (chalk) has given agglomeration and mixing problems. Sodium and potassium sulphate with hydration higher than two moles of water have given deliquescence problems. Said deliquescence problems have also been given by magnesium chloride and calcium chloride, both anhydrous and hydrates.

The concentration of the saline component ranges from 25% to 75% in composition weight; the concentration of active substance ranges from 2.5% and 25% in composition weight and the concentration of essential oils and/or camphor ranges from 2.5% and 25% in composition weight.

Compositions have been obtained by means of accurate physical mixing of components. Considering that menthol dissolves completely in the essences that are eventually added, mixing must be continued until a fluid crystalline powder is obtained. The attempt to eliminate physical mixing by providing active substances dissolved in a suitable solvent has given a negative result. The stability of the mixture obtained has proved excellent, as well as the compatibility of salts and essential oils with the active substance.

Following are some examples (expressed in weight percentages) of refreshing compositions:

### Example 1

| | |
|---|---|
| Sodium chloride | 61.5 |
| Anhydrous sodium sulphate | 34.0 |
| Menthol | 3.5 |
| Eucalyptol | 1.0 |

### Example 2

| | |
|---|---|
| Sodium chloride | 65.0 |
| Anhydrous sodium sulphate | 12.5 |
| Heptahydrate magnesium sulphate | 12.5 |
| Menthol | 10.0 |

### Example 3

| | |
|---|---|
| Potassium chloride | 10.5 |
| Sodium chloride | 30.0 |
| Heptahydrate magnesium sulphate | 15.0 |
| Menthol | 30.0 |
| Camphor | 15.0 |

### Example 4

| | |
|---|---|
| Sodium chloride | 50.0 |
| Anhydrous sodium sulphate | 15.0 |
| Menthol | 17.5 |
| Essence of pinus pumilio | 17.5 |

### Example 5

| | |
|---|---|
| Sodium chloride | 58.5 |
| Menthol | 24.0 |
| Lemon essential oil | 17.5 |

### Example 6

| | |
|---|---|
| Sodium chloride | 56.0 |
| Menthol | 28.0 |
| Camphor | 16.0 |

### Example 7

| | |
|---|---|
| Eucalyptol essential oil | 75.0 |
| Menthol | 25.0 |

Although not shown in the examples, the composition can also comprise a mineral regulator to regulate the intensity and duration of the freshness sensation. Said mineral regulator is ventilated talcum or pyogenic silica, which are available on the market with trademarks Aerosil™ or Levilite™. The mineral regulator is added in concentration ranging from 5 and 15% of composition weight.

Following is a description of some examples of composition formulations for an article with warming effect.

The composition consists in a mixture of salts and active substance with warming effect.

The active substance with warming effect that has proved to be most suitable is methyl nicotinate (CAS 93-60-7). Other nicotinates, in particular benzyl nicotinate, have not given a reliable response and have caused some problems of dispersion and irritation due to low melting point. In fact, these nicotinates are transformed in liquids at skin temperature.

In this case we have used the same saline component used in the composition with refreshing effect.

A mineral is added in the composition with warming effect to regulate the intensity and duration of the warming effect. The mineral that has give the best results is powdered colloidal sulphur that, due to absorption effects, seems to act as regulator in the production of warmth and at the same time as concentrator of efficacy.

The concentration of the saline component ranges from 45 to 85% in composition weight; the concentration of active substance with warming effect ranges from 2.5 and 10% in composition weight; and the concentration of mineral regulator ranges from 5 and 15% in composition weight.

Also in this case physical mixing of the various components is made without the use of diluents. The stability of the mixture obtained has proved excellent, as well as the compatibility of salts and mineral regulator with the active substance.

Following are some examples (expressed in weight percentages) of warming compositions:

### Example 1

| | |
|---|---|
| Sodium chloride | 61.5 |
| Anhydrous sodium sulphate | 20.0 |
| Powdered colloidal sulphur | 15.0 |
| Methyl nicotinate | 3.5 |

### Example 2

| | |
|---|---|
| Sodium chloride | 56.0 |
| Anhydrous sodium sulphate | 12.5 |
| Heptahydrate magnesium sulphate | 12.5 |
| Powdered colloidal sulphur | 15.0 |
| Methyl nicotinate | 4.0 |

### Example 3

| | |
|---|---|
| Potassium chloride | 10.0 |
| Sodium chloride | 58.0 |
| Heptahydrate magnesium sulphate | 15.0 |
| Powdered colloidal sulphur | 10.0 |
| Methyl nicotinate | 7.0 |

### Example 4

| | |
|---|---|
| Lavender essential oil | 97.0 |
| Camphor | 2.0 |
| Methyl nicotinate | 1.0 |

### Example 5

| | |
|---|---|
| Florentine iris fragrance | 97.0 |
| Camphor | 2.0 |
| Methyl nicotinate | 1.0 |

### Example 6

| | |
|---|---|
| Sodium chloride | 86.0 |
| Powdered colloidal sulphur | 9.0 |
| Methyl nicotinate | 5.0 |

In order to obtain a synergic effect of heat transfer to the composition, powdered Capsicum annuus (hot pepper) was added with concentration ranging from 2.5 and 10% in composition weight.

According to a first embodiment of the invention, the refreshing/warming compositions obtained in the preceding examples are arranged in a container or bag. The bag is preferably made of fabric or non-woven fabric of natural fibres (cotton, flax, jute, hemp, cellulose, and the like).

Numerous tests using bags of synthetic fibres (polyamides, polysaccharides, and polyethylene) have given negative results, causing irritation in the contact area and lower efficacy.

The best results have been obtained with a bag of cellulose fibre. Surprisingly, no negative effects are found if the bag of cellulose fibre that contains the composition is arranged in an external container of synthetic fibre (i.e.: a bag of cellulose fibre containing the composition arranged inside a collar of elasticated fabric that acts as support).

Bags of other natural fabrics of animal origin, such as wool and silk, have given negative results, obtaining unpredictable results and/or results not reproducible with reliability because of the protein structure of said fibres. Likewise, bags of natural leather or nappa leather have given negative results.

Surprisingly, regenerated leather has shown the same behaviour as cellulose fibres, giving positive results.

The above considerations have resulted in a second embodiment of the invention, wherein the composition has been directly incorporated in the mixture to produce sheets of regenerated leather, adding the composition in tanks together with components used to obtain the sheet of regenerated leather.

A support of regenerated leather that incorporates one of the compositions of the invention has shown a refreshing or warming effect that is slightly lower than a bag that contains said composition. Such behaviour is explained by the presence of cellulose glue contained in the binder used for regenerated leather.

A thin layer of regenerated leather without composition is applied on said support of regenerated leather containing the composition of the invention, which is designed to come in contact with the user's skin in order to prevent the direct contact of the composition with the skin.

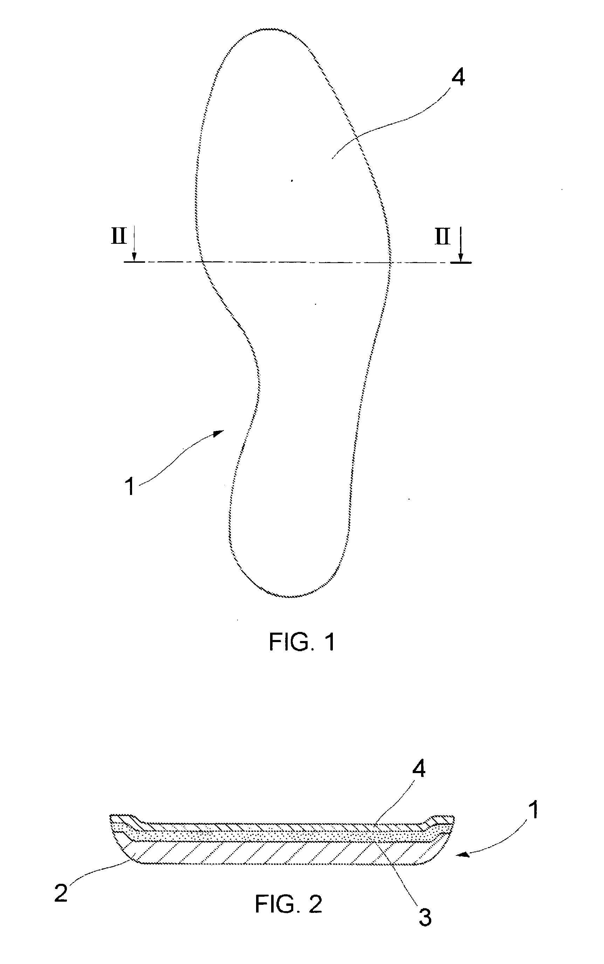

Such a solution is especially suitable for the realisation of an insole for shoes, such as the one illustrated in Figs. 1 and 2 and generally indicated with numeral (1).

The insole (1) is made of three layers: a lower layer (2), an intermediate layer (3) and an upper layer (4).

The lower layer (2) can be made of latex, polyethylene, polyester, texon, leather, EVA, felt, polyurethane, foam rubber. The lower layer (2) has thickness from 1 to 3 cm.

The intermediate layer (3) is made of regenerated leather or fabric soaked with the refreshing or warming composition of the invention. The intermediate layer (3) has thickness from 0.5 to 1.5 mm.

The upper layer (4) can be made of regenerated leather, leather, synthetic material or fabric to prevent the composition contained in the intermediate layer (3) from coming in direct contact with the foot plant. The upper layer (4) has thickness from 0.2 to 5 mm.

The three layers (2, 3, 4) are bound with ordinary glue or water-based latex.

In case of an insole with multiple layers, such as for sports shoes, shoes with arch supports and women's shoes with especially high heel, the layers of leather or fabric soaked with the composition can have different thickness and hardness. Some of the layers may also be of material other than leather or fabric, as long as the composition is in direct contact with another layer of fabric, leather, or synthetic material, and not with the skin.

In addition to insoles for shoes, various applications have been realised with such a solution, such as straps and wristbands, straps or bands for infrascapular area, headbands, as well as seat covers and boots.

According to a first variant of the second embodiment, the support can be made of polyester that incorporates said composition by means of mixing. According to a second variant of the second embodiment of the invention, the support is made of 100% cotton fabric in which said composition is incorporated by means of spraying.

Numerous variations and modifications can be made to the present embodiments of the invention by an expert of the field, while still falling within the scope of the invention as claimed in the enclosed claims.

## Claims

1. Insole (1) for shoes comprising a lower layer (2), an intermediate layer (3) and an upper layer (4) bounded each others with glue or water-based latex, wherein the intermediate layer (3) has thickness from 0.5 to 1.5 mm and is made of regenerated leather or fabric soaked containing a composition comprising:
- mineral salts,
- an active substance with refreshing or warming effect, said refreshing active substance comprising menthol (CAS 89-78-1) and said warming active substance comprising methyl nicotinate (CAS 93-60-7),
- camphor and/or essential oils,
said upper layer (4) having thickness from 0.2 to 5 mm to prevent the composition contained in the intermediate layer (3) from coming in direct contact with the foot plant.

2. Insole (1) according to claim 1 **characterised in that** said intermediate layer (3) is made of 100% cotton fabric, in which said composition is incorporated by means of spraying.

3. Insole (1) according to claim 1, **characterised in that** said intermediate layer (3) is made of regenerated leather in which said composition is mixed in the leather mix.

4. Insole (1) according to anyone of the preceding claims, **characterised in that** said upper layer (4) is made of regenerated leather, leather, synthetic material or fabric.

5. Insole (1) according to anyone of the preceding claims, **characterised in that** said lower layer (2) is made of latex, polyethylene, polyester, texon, leather, EVA, felt, polyurethane, foam rubber.

6. Insole (1) according to any one of the preceding claims, **characterised in that** the salts of said composition comprise sodium chloride salts.

7. Insole (1) according to anyone of the preceding claims, **characterised in that** said composition has a concentration of saline component from 25 and 75% with respect to the weight of the composition and concentration of active substance from 2.5 and 25% with respect to the weight of the composition.

8. Insole (1) according to anyone of the preceding claims, **characterised in that** said composition also comprises a mineral regulator composed of talcum or pyogenic silica or powdered sulphur in concentration from 5 to 15% with respect to the weight of the composition.

9. Insole (1) according to anyone of the preceding claims, **characterised in that** said composition also comprises powdered Capsicum annuus with concentration from 2.5 and 10% with respect to the weight of the composition.

## Patentansprüche

1. Sohle (1) für Schuhe umfassend eine Unterschicht (2), eine Zwischenschicht (3) und eine Oberschicht (4), die mittels Klebstoff oder Latex auf Wasserbasis miteinander verbunden sind, wobei die Zwischenschicht (3) eine Dicke zwischen 0,5 und 1,5 mm aufweist und aus wiederaufbereitetem Leder oder imprägniertem Stoff besteht mit einer Zusammensetzung umfassend:
- Mineralsalze,
- einen Wirkstoff mit kühlender oder wärmender Wirkung, wobei der kühlende Wirkstoff Menthol (CAS 89-78-1) und der wärmende Wirkstoff Methylnicotinat (CAS 93-60-7) umfasst,
- Campher und/oder essentielle Öle,
wobei die Oberschicht (4) eine Dicke zwischen 0,2 und 5 mm aufweist, um zu verhindern, dass die in der Zwischenschicht (3) enthaltene Zusammensetzung in unmittelbaren Kontakt mit der Fußsohle gelangt.

2. Sohle (1) gemäß Anspruch 1, **dadurch gekennzeichnet, dass** die Zwischenschicht (3) zu 100% aus Baumwollstoff besteht, in den diese Zusammensetzung eingespritzt wird.

3. Sohle (1) gemäß Anspruch 1, **dadurch gekennzeichnet, dass** die Zwischenschicht (3) aus wiederaufbereitetem Leder besteht, wobei diese Zusammensetzung unter die Ledermischung gemischt wird.

4. Sohle (1) nach einem beliebigen der vorstehenden Ansprüche, **dadurch gekennzeichnet, dass** die Oberschicht (4) aus wiederaufbereitetem Leder, Leder, Synthetikmaterial oder Stoff hergestellt ist.

5. Sohle (1) nach einem beliebigen der vorstehenden Ansprüche, **dadurch gekennzeichnet, dass** die Unterschicht (2) aus Latex, Polyethylen, Polyester, Texon, Leder, EVA, Filz, Polyurethan oder Schaumgummi hergestellt ist.

6. Sohle (1) nach einem beliebigen der vorstehenden Ansprüche, **dadurch gekennzeichnet, dass** die Salze der Zusammensetzung Natriumchloridsalze umfassen.

7. Sohle (1) nach einem beliebigen der vorstehenden Ansprüche, **dadurch gekennzeichnet, dass** die Zusammensetzung eine Konzentration des Salzanteils zwischen 25 und 75 Gew.-% der Zusammensetzung und eine Konzentration des Wirkstoffs zwischen 2,5 und 25 Gew.-% der Zusammensetzung aufweist.

8. Sohle (1) nach einem beliebigen der vorstehenden Ansprüche, **dadurch gekennzeichnet, dass** die Zusammensetzung außerdem einen Mineralreglerstoff umfasst, der aus pyrogener Kieselsäure oder Talkum oder Schwefelpulver in einer Konzentration zwischen 5 und 15 Gew.-% der Konzentration besteht.

9. Sohle (1) nach einem beliebigen der vorstehenden Ansprüche, **dadurch gekennzeichnet, dass** die Zusammensetzung außerdem Capsicum annuum in Pulverform in einer Konzentration zwischen 2,5 und 10 Gew.-% der Zusammensetzung umfasst.

## Revendications

1. Semelle (1) pour chaussures comprenant une couche inférieure (2), une couche intermédiaire (3) et une couche supérieure (4) liées entre elles avec de la colle ou du latex à base aqueuse, où la couche intermédiaire (3) a une épaisseur comprise entre 0.5 et 1.5 mm et elle est réalisée en synderme contenant une composition comprenant :
- des sels minéraux
- une substance active à effet rafraîchissant ou réchauffant, la dite substance active rafraîchissante comprenant du menthol (CAS 89-78-1) et la dite substance active réchauffante comprenant du nicotinate méthylique (CAS 93-60-7),
- camphre et/ou huiles essentielles,
la dite couche supérieure (4) ayant une épaisseur comprise entre 0.2 et 5 mm de manière à éviter que la composition contenue dans la couche intermédiaire (3) soit en contact direct avec la plante du pied.

2. Semelle (1) selon la revendication 1, caractérisée en que la dite couche intermédiaire (3) est du tissu 100% coton, où la dite composition est englobée moyennant projection.

3. Semelle (1) selon la revendication 1, caractérisée en que la dite couche intermédiaire (3) est du synderme dans lequel la dite composition est imprégnée dans le mélange du cuir.

4. Semelle (1) selon l'une quelconque des revendications précédentes, **caractérisée en ce que** la dite couche supérieure (4) est réalisée en synderme, cuir, matériel synthétique ou tissu.

5. Semelle (1) selon l'une quelconque des revendications précédentes, **caractérisée en ce que** la dite couche inférieure (2) est réalisée en latex, polyéthylène, polyester, texon, cuir, EVA, feutre, polyuréthane, caoutchouc mousse.

6. Semelle (1) selon l'une quelconque des revendications précédentes, **caractérisée en ce que** les sels de la dite composition comprennent des Sels de chlorure de sodium.

7. Semelle (1) selon l'une quelconque des revendications précédentes, **caractérisée en ce que** la dite composition a une concentration de la composante saline comprise entre le 25 et le 75 % par rapport au poids de la composition et une concentration de la substance active comprise entre le 2,5 et le 25 % par rapport au poids de la composition.

8. Semelle (1) selon l'une quelconque des revendications précédentes, **caractérisée en ce que** la dite composition comprend en outre un régulateur minéral constitué de talc ou de silice pyogène ou de poudre de soufre, dans une concentration comprise entre le 5 et le 15% par rapport au poids de la composition.

9. Semelle (1) selon l'une quelconque des revendications précédentes, **caractérisée en ce que** la dite composition comprend également des piments du genre 'Capsicum annuus' en poudre, dans une concentration comprise entre le 2,5 et le 10% par rapport au poids de la composition.
